(19) Europäisches Patentamt
European Patent Office
Office européen des brevets

(11) EP 3 733 782 A1

(12) # EUROPEAN PATENT APPLICATION
published in accordance with Art. 153(4) EPC

(43) Date of publication:
**04.11.2020 Bulletin 2020/45**

(21) Application number: **18894424.3**

(22) Date of filing: **28.12.2018**

(51) Int Cl.:
***C08L 101/12*** *(2006.01)* ***C08F 2/12*** *(2006.01)*
***C08L 25/06*** *(2006.01)* ***C09K 11/06*** *(2006.01)*
***G01N 33/533*** *(2006.01)* ***G01N 33/553*** *(2006.01)*

(86) International application number:
**PCT/JP2018/048622**

(87) International publication number:
**WO 2019/132045 (04.07.2019 Gazette 2019/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.12.2017 JP 2017254640**

(71) Applicants:
- **SEKISUI CHEMICAL CO., LTD.**
**Osaka-shi**
**Osaka**
**530-8565 (JP)**
- **Sekisui Medical Co., Ltd.**
**Tokyo 103-0027 (JP)**

(72) Inventors:
- **YAMAGAMI Mai**
**Mishima-gun, Osaka 618-0021 (JP)**
- **OBINATA Shuhei**
**Mishima-gun, Osaka 618-0021 (JP)**
- **WAKIYA Takeshi**
**Mishima-gun, Osaka 618-0021 (JP)**
- **YAJI Maasa**
**Tokyo 103-0027 (JP)**

(74) Representative: **Hoffmann Eitle**
**Patent- und Rechtsanwälte PartmbB**
**Arabellastraße 30**
**81925 München (DE)**

(54) **METAL COMPLEX PARTICLES AND IMMUNOASSAY REAGENT USING SAME**

(57) Metal complex particle including a magnetic material and a particle size adjusting composition containing a hydrophobic polymer, with the magnetic material including a metal complex consisting of a metal ion and a ligand and having both magnetism and fluorescence, and a content of the particle size adjusting composition in the metal complex particle being less than 60 w%.

EP 3 733 782 A1

## Description

### Technical Field

The present invention relates to metal complex particles and an immunoassay reagent using the same.

### Background Art

Conventionally, methods for measuring or purifying a protein or the like in a sample containing a biological substance, for example, in a biological sample have been known. Examples thereof include a method in which a protein or the like in a biological sample is allowed to bind to the surface of particles capable of binding the protein, the particles are washed so as to remove impurities other than the target protein or the like in the biological sample, and the particles to which the protein or the like binds are collected to measure a binding amount of the protein and a method in which a protein is dissociated in a protein dissociation solution to purify the protein.

In addition, a method in which a target protein is detected by acting an antibody labeled with an enzyme on the target protein (antigen, etc.) and then optically measuring light emitted through a reaction between the enzyme and a luminescent reagent (ELISA) has been widely utilized.

Patent Literatures 1 and 2 described below disclose that particles having magnetism are used for the above-described ELISA as such particles can be easily separated and collected by virtue of magnetic force.

Further, particles enabling fluorescent labeling by imparting fluorescence to particles having magnetism have been proposed. For example, Patent Literature 3 discloses particles to which magnetism and fluorescence are imparted by impregnating a polymer layer formed on ferrite particle surfaces with a europium chelate complex.

In addition, Patent Literature 3 describes as methods for obtaining an aqueous suspension in which a europium chelate complex is embedded into magnetic particles, (1) although no specific example is shown, a method for obtaining an aqueous suspension of europium chelate complex-containing magnetic particles by adding a low boiling point nonaqueous solvent (preferably, acetone) solution in which a europium chelate complex is dissolved and which has high affinity with water to an aqueous suspension of polymer-coated magnetic particles to swell polymer layers and simultaneously allow the europium chelate complex to enter therein, and subsequently evaporating the solvent and (2) a method for obtaining an aqueous suspension of europium chelate complex-containing magnetic particles by adding slightly crosslinked polymer-coated magnetic particles from which water has been removed to an acetone solution in which a europium chelate complex is dissolved to swell polymer layers and simultaneously allow the europium chelate complex to enter therein, and subsequently evaporating acetone and adding water.

Further, Patent Literature 4 describes (3) a method for obtaining an aqueous suspension of europium chelate complex-containing magnetic particles by adding slightly crosslinked polymer-coated magnetic particles from which water has been removed through alcohol washing or the like to an acetone solution in which a europium chelate complex is dissolved to swell polymer layers and simultaneously allow the europium chelate complex to enter therein, and subsequently adding water to evaporate acetone.

### Citation List

### Patent Literature

Patent Literature 1
Japanese Patent Laid-Open No. 2005-062087
Patent Literature 2
International Publication No. WO 2012-173002
Patent Literature 3
Japanese Patent Laid-Open No. 2008-127454
Patent Literature 4
International Publication No. WO 2010-029739


### Summary of Invention

### Technical Problem

However, there has been a problem in Patent Literature 3 as follows. Since magnetic particles obtained by the

described production method have a maximum diameter of 300 nm, amounts of a fluorescent material and a magnetic material contained in one particle are small and speed of separation at separation using a magnet is slow. In addition, ferrite absorbs fluorescence. Therefore, sufficient fluorescence cannot be obtained and sensitivity is low. Further, Patent Literature 3 shows no specific example of the method according to (1), and when acetone, which is said to be preferable, is used, the europium complex precipitates once the acetone solution of the europium chelate complex is added to the polymer-coated magnetic particle aqueous suspension, and it has been difficult to allow the europium chelate complex to enter polymer layers at a required amount. When acetone is used in a large amount relative to water so that no europium chelate complex precipitates, the concentration of the europium chelate complex decreases and the europium chelate complex cannot be sufficiently contained. In the method according to (2), particles coalesce with each other during the step of evaporating acetone and it has been difficult to obtain single particles. In the method according to (3), the chelate complex also precipitates at the time of adding water and it has been extremely difficult to remove the precipitate.

As described above, particles having highly sensitive magnetism and fluorescence, which can be promptly separated/collected by a magnetic and which enable fluorescent labeling, are not practically used as an immunoassay reagent.

The present invention aims at providing metal complex particles excellent in magnetic collectiveness and having both magnetism and fluorescence and providing an immunoassay reagent using the same.

Solution to Problem

The present invention relates to the following contents.

(1) Metal complex particle including a magnetic material and a particle size adjusting composition containing a hydrophobic polymer, wherein the magnetic material includes a metal complex consisting of a metal ion and a ligand and having both magnetism and fluorescence, and a content of the particle size adjusting composition in the metal complex particle is less than 60 w%.
(2) The metal complex particle according to (1), wherein the magnetic material is free of a metal complex not having fluorescence but having magnetism.
(3) The metal complex particle according to (1), wherein the magnetic material consists of the metal complex.
(4) The metal complex particle according to any one of (1) to (3), wherein a magnetic moment of the metal ion forming the metal complex is 7.5 to 10.7.
(5) The metal complex particle according to any one of (1) to (4), wherein the metal ion forming the metal complex is at least one selected from the group consisting of ions of terbium, dysprosium, holmium, and erbium.
(6) The metal complex particle according to any one of (1) to (5), wherein the ligand forming the metal complex is both of or any one of a β-diketone ligand and a heterocyclic ligand.
(7) The metal complex particle according to any one of (1) to (6), wherein the ligand forming the metal complex is selected from (1) dibenzoylmethane or thenoyl fluoroacetone for the β-diketone ligand and (2) 2,2'-bipyridyl, 2,2':6,2''-terpyridine, or 1,10-phenanthroline for the heterocyclic ligand.
(8) The metal complex particle according to any one of (1) to (7), wherein an average particle size of the metal complex particle is 1 μm to 5 μm.
(9) The metal complex particle according to any one of (1) to (8), wherein a content of the metal complex in the metal complex particle is 40 w% to 98 w%.
(10) The metal complex particle according to any one of (1) to (9), having a substance for carrying on the surface thereof.
(11) The metal complex particle according to any one of (1) to (10), wherein the substance for carrying is an antibody or an antigen that specifically reacts with a detection object substance.
(12) An immunoassay reagent including the metal complex particle according to (10).

Advantageous Effect of Invention

According to the present invention, metal complex particles excellent in magnetic collectiveness and having both magnetism and fluorescence and an immunoassay reagent using the same are provided.

Brief Description of Drawings

[Figure 1] Figure 1A is a diagram schematically illustrating metal distribution in a cross-section of a particle, and

Figure 1B is a partially enlarged view schematically illustrating metal distribution in a cross-section of a particle.
[Figure 2] Figure 2A and Figure 2B are diagrams each schematically illustrating metal distribution in a cross-section of a particle according to a related art.

Description of Embodiments

The present inventors conducted intensive studies to solve the above-described problem. As a result, the present inventors have found that there are several kinds of metal complexes each having both magnetism and fluorescence among multiple rare earth complexes. Then, the present inventors have found that metal complex particles excellent in magnetic collectiveness and having a fluorescence emitting property are obtained by preparing particles of these metal complexes with a uniform particle size distribution. Further, high magnetic collectiveness and improved measurement accuracy (visibility) are achieved by using the obtained particles for an immunoassay reagent. Consequently, the above-described problem has been solved. Hereinafter, the present invention will be described by giving embodiments. However, the present invention is not limited to the following embodiments.

[Metal complex particles]

The present invention relates to a metal complex particle including a magnetic material and a particle size adjusting composition containing a hydrophobic polymer, the magnetic material including a metal complex consisting of a metal ion and a ligand and having both magnetism and fluorescence, and a content of the particle size adjusting composition in the metal complex particles being less than 60 w%. It is preferable that the metal complex particle contains the metal complex as a sole substance having magnetism.

Since the content of the metal complex having magnetism is high, the metal complex particles of the present invention have high magnetic collectiveness, and a quantity of magnetism per particle becomes uniform at the same time. Therefore, responses to magnetic force of individual particles become uniform at separation using magnetic force, and variations during separation become small. Further, since the metal complex has a fluorescent property besides magnetism, detection sensitivity is enhanced by using these metal complex particles for an immunoassay reagent. In addition, the fluorescent spectrum of the metal complex particles can be changed by changing the kind of the metal complex, and color of the metal complex particles is changed. Therefore, wrong diagnoses resulting from a mix-up of reagents or the like at the time of measuring plural specimens can be avoided.

Since magnetism and fluorescence of the metal complex particles of the present invention are exhibited by the same metal complex as compared with particles separately containing a magnetic material and a fluorescent material, there is no negative mutual interaction between a magnetism-exhibiting substance and a fluorescence-exhibiting substance, and both of magnetism and fluorescence can be effectively exhibited. In addition, since the metal complex particles of the present invention enable the metal complex to be highly contained, high magnetic collectiveness can be exhibited. Further, since particle sizes of the metal complex particles of the present invention are uniform, responses to a magnetic become also uniform. Therefore, uniform magnetic collectiveness is exhibited.

The above-described particle size adjusting composition mainly contains a polymer (hereinafter, also simply referred to as "polymer") obtained through polymerization (including polycondensation) of a polymerizable monomer (hereinafter, also simply referred to as "monomer"). The above-described polymer includes a copolymer of one kind or two or more kinds of polymerizable monomers.

Examples of the above-described polymer include a polymer of one or more kinds of polymerizable monomers including (un)saturated hydrocarbons, aromatic hydrocarbons, (un)saturated fatty acids, aromatic carboxylic acids, (un)saturated ketones, aromatic ketones, (un)saturated alcohols, aromatic alcohols, (un)saturated amines, aromatic amines, (un)saturated thiols, aromatic thiols, and organosilicon compounds.

Examples of the above-described polymer include polyolefins such as polyethylene, polypropylene, polybutylene, polymethyl pentene, and polybutadiene; polyethers such as polyethylene glycol, polypropylene glycol, and tetramethylene glycol; polystyrenes; poly(meth)acrylic acids; poly(meth)acrylic acid esters; polyvinyl alcohols; polyvinyl esters; polyvinyl ethers; phenol resins; melamine resins; benzoguanamine resins; allyl resins; furan resins; (un)saturated polyesters; epoxy resins; polysiloxanes; polyamides; polyimides; polyurethanes; Teflon (R); acrylonitrile/styrene resins; styrene/butadiene resins; halogenated vinyl resins; polycarbonates; polyacetals; polyether sulfones; polyphenylene oxides; polyetheretherketones; sugars; starches; cellulose; polypeptides; and a polymer and the like obtained by polymerizing one kind or two or more kinds of various polymerizable monomers having a polymerizable functional group. These polymers may be used singly, or two or more thereof may be used in combination.

The above-described polymer is produced through radical polymerization, ionic polymerization, coordination polymerization, ring-opening polymerization, isomerization polymerization, cyclopolymerization, elimination polymerization, polyaddition, polycondensation, and addition condensation.

One kind of the above-described polymerizable monomer may be used singly or two or more kinds thereof

may be used in combination at any ratio. In addition, when two or more kinds of the polymerizable monomer are used, the combination of the two or more kinds of the polymerizable monomer may be any of a combination of two or more kinds of monofunctional polymerizable monomers, a combination of two or more kinds of polyfunctional polymerizable monomers, and a combination of one or more kinds of monofunctional monomers and one or more kinds of polyfunctional monomers. Molecular weights of these polymers are 80 to 30,000, for example, but are not particularly limited. In the present invention and the present description, the molecular weight of a polymer refers to a weight average molecular weight measured in terms of polystyrene using gel permeation chromatography (GPC). The weight average molecular weight described later is a value measured by the following measurement.
GPC apparatus: APC system (manufactured by Waters Corporation)
Column: HSPgel HR MB-M (manufactured by Waters Corporation) 6.0 × 150 mm
Eluent: Tetrahydrofuran (THF), flow rate: 0.5 mL/min, column temperature: 40°C, injection amount: 10 μL, detector: RI

The above-described polymerizable monomer (hereinafter, also simply referred to as "monomer") is not particularly limited as long as it is a compound containing one or more polymerizable functional groups within one molecule. The monomer may be a monofunctional monomer containing one polymerizable functional group within one molecule or may be a polyfunctional monomer containing two or more polymerizable functional groups within one molecule. In addition, the monomer may be an oligomer or a prepolymer which contains one or more polymerizable functional groups within one molecule, or the like.

The above-described polymerizable functional group may be a radically polymerizable functional group, an ionically polymerizable functional group, or a coordination-polymerizable functional group, and a radically polymerizable functional group is preferable. Polymerizable groups such as an ethylenically unsaturated bond-containing group, epoxy group, oxetane group, and methylol group can be exemplified in view of reactivity of polymerization reaction, and an ethylenically unsaturated group is more preferable. Examples of the ethylenically unsaturated group can include (meth)acryloyloxy group, (meth)acryloyl group, vinyl group, an aromatic vinyl group, and allyl group, and (meth)acryloyloxy group, vinyl group, and an aromatic vinyl group are more preferable. The "(meth)acryloyl group" described in the present invention and the present description is used in referring to at least one of acryloyl group and methacryloyl group. The same is applied to the "(meth)acryloyloxy group," "(meth)acrylate," "(meth)acryl," and the like. With respect to the polyfunctional monomer, the number of polymerizable groups contained in a compound is two or more per molecule.

Specific examples of the monofunctional monomer having the above-described ethylenically unsaturated group are not particularly limited.

Examples of the monofunctional monomer having (meth)acryloyl group include (meth)acrylate monomers such as methyl (meth)acrylate, ethyl (meth)acrylate, propyl (meth)acrylate, butyl (meth)acrylate, 2-ethylhexyl (meth)acrylate, lauryl (meth)acrylate, cetyl (meth)acrylate, stearyl (meth)acrylate, cyclohexyl (meth)acrylate, isobornyl (meth)acrylate, 2-hydroxyethyl (meth)acrylate, glycerol (meth)acrylate, polyoxyethylene (meth)acrylate, glycidyl (meth)acrylate, trifluoromethyl (meth)acrylate, pentafluoroethyl (meth)acrylate, 3-(meth)acryloxypropyl methyldimethoxysilane, 3-(meth)acryloxypropyl trimethoxysilane, 3-(meth)acryloxypropyl methyldiethoxysilane, and 3-(meth)acryloxypropyl triethoxysilane.

Examples of the monofunctional monomer having vinyl group include olefin monomers such as diisobutylene, isobutylene, Linealene, ethylene, and propylene; conjugated diene monomers such as isoprene and butadiene; vinyl ether monomers such as methyl vinyl ether, ethyl vinyl ether, and propyl vinyl ether; vinyl ester monomers such as vinyl acetate, vinyl butyrate, vinyl laurate, and vinyl stearate; halogen-containing vinyl monomers such as vinyl chloride and vinyl fluoride; nitrile-containing vinyl monomers such as (meth)acrylonitrile; silane alkoxide-containing vinyl monomers such as vinyltrimethoxysilane, vinyltriethoxysilane, dimethoxymethylvinylsilane, dimethoxyethylvinylsilane, diethoxymethylvinylsilane, diethoxyethylvinylsilane, methylvinyldimethoxysilane, ethylvinyldimethoxysilane, methylvinyldiethoxysilane, and ethylvinyldiethoxysilane.

Examples of the monofunctional monomer having an aromatic vinyl group include aromatic vinyl monomers such as styrene, α-methyl styrene, chlorostyrene, vinylnaphthalene, 4-vinylbiphenyl, and p-styryltrimethoxysilane.

Specific examples of the polyfunctional monomer having the above-described ethylenically unsaturated group are not particularly limited.

Examples of the polyfunctional monomer having (meth)acryloyl group include polyfunctional (meth)acrylate monomers such as tetramethylolmethanetetra(meth)acrylate, tetramethylolmethanetri(meth)acrylate, tetramethylolmethanedi(meth)acrylate, trimethylolpropanetri(meth)acrylate, dipentaerythritol hexa(meth)acrylate, dipentaerythritol penta(meth)acrylate, glycerol tri(meth)acrylate, glycerol di(meth)acrylate, (poly)ethylene glycol di(meth)acrylate, (poly)propylene glycol di(meth)acrylate, (poly)tetramethylene glycol di(meth)acrylate, 1,4-butanediol di(meth)acrylate, and 9,9-bis[4-(2-acryloyloxyethyloxy)]phenylfluorene.

Examples of the polyfunctional monomer having vinyl group include 1,4-divinyloxybutane, divinyl sulfone, 9,9'-bis(4-allyloxyphenyl)fluorene, and ethylmethyldivinylsilane.

Examples of the polyfunctional monomer having an aromatic vinyl group include aromatic vinyl monomers such as divinylbenzene, divinylnaphthalene, and divinylbiphenyl.

Examples of the polyfunctional monomer having allyl group include allyl monomers such as triallyl (iso)cyanurate, triallyl trimellitate, diallyl phthalate, diallyl acrylamide, diallyl ether, 9,9'-bis(4-allyloxyphenyl)fluorene.

The above-described particle size adjusting composition preferably contains a hydrophobic polymer. In the present description, the "hydrophobic polymer" refers to a polymer having a solubility in water at 25°C of 5 w% or less and includes a block polymer which has a hydrophilic functional group or has a hydrophilic block segment. In addition, a polymer which may be dispersed in water at a predetermined probability is also included in the hydrophobic polymer.

The magnetic material is preferably a metal complex consisting of a metal ion and a ligand. The metals and ligands listed below are preferably used as a metal and a ligand constituting the metal complex.

The metal constituting the metal complex is not particularly limited as long as it exhibits a magnetic property and a fluorescent property as a metal complex and the ligand described later binds thereto. However, a metal having a magnetic moment of 7.5 or more is preferable so as to achieve high magnetic collectiveness as metal complex particles. Further, the magnetic moment is preferably 9.0 or more. Examples of the metal having a magnetic moment of 7.5 or more include terbium (Tb), dysprosium (Dy), holmium (Ho), erbium (Er), and thulium (Tm). Among them, Tb, Dy, Ho, Er, and the like are especially preferable because their magnetic moments are 9.0 or more. These metals may be used singly, or two or more thereof may be used in combination.

The ligand forming the metal complex is not particularly limited as long as it forms a complex with the above-described metal and exhibits magnetism and fluorescence. However, a β-diketone ligand represented by Formula (X), a heterocyclic ligand, and a phosphorus hydrophobic neutral ligand are preferable because these ligands likely to form metal complex particles by the hydrophobic interaction with the above-described particle size adjusting composition, especially with the hydrophobic polymer in the above-described particle size adjusting composition. These ligands may be used singly, or two or more thereof may be used in combination. It is especially preferable to use a β-diketone ligand and a heterocyclic ligand in combination or use a heterocyclic ligand so as to obtain more strong magnetism and fluorescence.

A ligand represented by the following Formula (X) can be used as the β-diketone ligand.

[Formula 1]

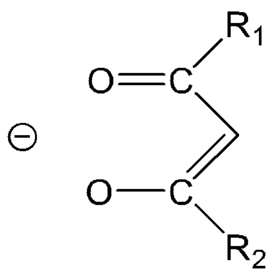

(X)

(In Formula (X) above, $R_1$ and $R_2$ may be the same or different and represent a thienyl group, a trifluoromethyl group, a fluoromethyl group, a furyl group, a phenyl group, a naphthyl group, a benzyl group, a ter-butyl group, a methyl group, or the like.)

Examples of such a β-diketone ligand include thenoyltrifluoroacetone, naphthoyltrifluoroacetone, benzoyltrifluoroacetone, furoyltrifluoroacetone, pivaloyltrifluoroacetone, hexafluoroacetylacetone, trifluoroacetylacetone, fluoroacetylacetone, dibenzoylmethane, dithenoylmethane, and furoyltrifluoroacetone. Among them, thenoyltrifluoroacetone (TTA) and dibenzoylmethane (DBM) are preferable.

Heterocyclic (anion having a hetero ring) ligand

Examples of the heterocyclic ligand which has a hetero ring containing a heteroatom such as a nitrogen atom or an oxygen atom include 2,2'-bipyridyl, 4,4'-dimethyl-2,2'-bipyridyl, 6,6'-dimethyl-2,2'-bipyridyl, 5,5'-dimethyl-2,2'-bipyridyl, 2,2'-bipyrimidyl, 4,4'-diethyl-2,2'-bipyridyl, 8-quinolinol, 2,2':6,2''-terpyridine, 4,4'-dinonyl-2,2'-bipyridyl, di-tert-butylbipyridine, 1,10-phenanthroline, 3,4,7,8-tetramethyl-1,10-phenanthroline, bathophenanthroline, 5,6-dimethyl-1,10-phenanthroline, phthalocyanine, 5,15-diphenyl porphyrin, tetraphenyl porphyrin, and 5,10,15,20-tetra(4-pyridyl)porphyrin, and 3-pyrazolone. Among them, 2,2'-bipyridyl, 2,2':6,2''-terpyridine, and 1,10-phenanthroline are preferable.

Phosphorus hydrophobic neutral ligand

Examples of the hydrophobic neutral ligand having a phosphor atom include trioctylphosphine oxide, tri-n-octylphosphine, and tributyl phosphate. Among them, trioctylphosphine oxide and tri-n-octylphosphine are especially preferable.

The content A (w%) of the metal complex in particles is calculated according to Formula (1) to Formula (3) from a residue remaining after decomposition of a polymer and a ligand when the particles are heated to 1000°C in an inert gas.
When Y (g) of holmium oxide having a molecular weight of 377.86 (g/mol) remains after weighing X (g) of particles and calcining the particles at 1000°C in air, the weight of substance Z (mol) of holmium oxide contained in the particles is calculated according to Formula (1).

$$Z\ (mol) = Y\ (g)/377.86\ (g/mol) \qquad \text{Formula (1)}$$

The amount a (g) of the metal complex contained in the particles is calculated according to Formula (2) with the molecular weight of the used complex being M (g/mol).

$$a\ (g) = M\ (g/mol) \times Z\ (mol) \qquad \text{Formula (2)}$$

Accordingly, the content A (w%) of the metal complex contained in the particles is calculated according to Formula (3).

$$A\ (w\%) = [a\ (g)/X\ (g)] \times 100 \qquad \text{Formula (3)}$$

In addition, the above-described residue can be obtained from an amount remaining after heating particles from 35°C to 1000°C at 5°C/min and keeping 1000°C for five minutes using TG-DTA 6300 (manufactured by Hitachi High-Technologies Corporation) apparatus.
One preferable mode of the metal complex particle is a metal complex particle including a styrene hydrophobic polymer or a (meth)acrylate hydrophobic polymer as the particle size adjusting composition and a metal complex of holmium ion, terbium ion, or dysprosium ion and at least one of a β-diketone ligand and a heterocyclic ligand or both ligands as the metal complex. The content of the metal complex is preferably 40 w% to 98 w% based on the total weight of the particles.
The content of the metal complex is preferably 40 w% to 98 w%, while depending on the kind thereof. When the content is 40 w% or more, sufficient magnetic collectiveness is exhibited. When the content is 98 w% or less, particulate shape can be maintained. The content of the metal complex is more preferably 50 w% or more and still more preferably 60 w% or more.
While the particle size of the metal complex particles of the present invention is note particularly limited, the particle size is preferably 500 nm to 10 μm so as to achieve high magnetic collectiveness. When the particle size is 500 nm or more, speed of separation using a magnet is high. In addition, when the particle size is 10 μm or less, particles are less likely to precipitate. The particles size is more preferably 1 μm to 5 μm.
An average particle size of particles was obtained by observing particles by a scanning type electron microscope and arithmetically averaging the maximum diameters of 50 particles arbitrarily selected from particles on the observed image.
The coefficient of variation (CV value) of the metal complex particles of the present invention is preferably 20% or less so that reproducibility in measurement results becomes good. When the coefficient of variation exceeds 20%, variations occur in magnetic collectiveness, and reproducibility in measurement results may decrease. The coefficient of variation is more preferably 10% or less.
The above-described coefficient of variation is obtained by observing particles by a scanning type electron microscope, obtaining a standard deviation of the particles from 50 particles arbitrarily selected from particles on the observed image, and obtaining the CV value of the particle sizes of the particles according to the following formula.

$$CV\ value\ (\%) = (\rho/Dn) \times 100$$

p: Standard deviation of particle sizes of particles
Dn: Average value of particle sizes of particles

The following production methods are examples of a production method of the metal complex particles of the present invention.

(1) A method conducted by A: a step of dissolving a particle size adjusting composition and a metal complex in a hydrophobic solvent capable of dissolving both of the particle size adjusting composition and the metal complex, B: a step of adding the solution obtained in step A to an aqueous solution in which a surfactant and/or a dispersion stabilizer is dissolved to form a droplet through emulsification, phase inversion emulsification, suspension, or the like, C: a step of distilling the hydrophobic solvent away by heating or under a reduced pressure, and D: a step of uniforming particle sizes through filtration, classification, or the like.

(2) A method conducted by A: a step of dissolving a polymerizable monomer, a hydrophobic polymerization initiator, and a metal complex, B: a step of forming a droplet through emulsification, suspension, or the like in an aqueous solution in which a surfactant and/or a dispersion stabilizer is dissolved, C: a step of polymerizing a particle size adjusting composition under the presence of a metal complex through heating and/or light irradiation, and D: a step of uniforming particle sizes through filtration, classification, or the like. A hydrophobic solvent capable of dissolving both of the polymerizable monomer and the metal complex may be added in step A as needed. In this case, a step of distilling the hydrophobic solvent away by heating or under a reduced pressure is added after step C. In addition, the polymerizable monomer may be the monofunctional monomer or the polyfunctional monomer described above, or both of the monofunctional monomer and the polyfunctional monomer may be used in combination.

(3) A: a step of dispersing particles (hereinafter also referred to as "template particles"), which are prepared from a particle size adjusting composition and which are to be a template, in water in which a surfactant and/or a dispersion stabilizer is dissolved, B: a step of preparing a metal complex solution in which a metal complex is dissolved in a hydrophobic organic solvent and/or a polymerizable monomer capable of dissolving both of the above-described template particles and the metal complex, C: a step of adding the metal complex solution obtained in step B to the dispersion liquid obtained in step A to allow the metal complex to enter the above-described template particles together with the hydrophobic organic solvent and/or the polymerizable monomer, and D: polymerizing the polymerizable monomer through heating and/or light irradiation and distilling the hydrophobic organic solvent away by heating or under a reduced pressure. A hydrophobic polymerization initiator may be added in step B. In addition, in the case where no polymerizable monomer is added in step B, the polymerization step in step D is not required.

The method according to (3) is preferably used because particle sizes of the metal complex particles obtained can be made uniform by using particles uniform in particle size for the particles (template particles) which are prepared from a particle size adjusting composition and which are to be a template and because the amount of the metal complex to be embedded can be made uniform.

As described above, the template particles prepared from the above-described particle size adjusting composition mainly contain a polymer obtained by polymerizing a polymerizable monomer, and said polymer includes a copolymer of one kind or two or more kinds of polymerizable monomers.

The polymer mainly constituting the above-described template particles is not particularly limited as long as it is a hydrophobic polymer, and a polymer obtained by polymerizing the above-described monofunctional polymerizable monomers through radical polymerization, ionic polymerization, coordination polymerization, or the like is used. Among others, a polymer/copolymer the polymerizable monomer of which mainly contains an aromatic vinyl monomer, a (meth)acrylate monomer, or a vinyl ester monomer is preferably used because of its high solubility in the hydrophobic solvent described later. An aromatic vinyl monomer and a (meth)acrylate monomer are more preferable.

Emulsion polymerization, suspension polymerization, soap-free polymerization, dispersion polymerization, miniemulsion polymerization, mircosuspension polymerization; phase inversion emulsification and solvent evaporation method using a polymer solution, and the like can be used for a production method of the above-described template particles. However, soap-free polymerization and dispersion polymerization are preferably used because particles uniform in particle size are obtained.

In the case of the above-described production method (1) or (3), the above-described polymerizable monomer and an initiator may be added, and polymerization may be performed through heating or the like. It is especially preferable when a polyfunctional monomer is used because a crosslinked structure can be introduced into the insides of particles. A polymer in which the above-described polymerizable monomer added has been polymerized through heating or the like is also referred to as a "second particle size adjusting composition," since such a polymer constitutes a part of the particle size adjusting composition.

An upper limit of the content of the particle size adjusting composition in the metal complex particles described above is preferably less than 60 w% and more preferably less than 50 w%. A lower limit is preferably 2 w% or more and more preferably 5 w% or more. When the content is 2 w% or more, a particle shape is likely to be maintained and outflow of the metal complex can be prevented. In addition, since the content of the metal complex is preferably 30 w% or more in order to exhibit sufficient magnetic collectiveness of the metal complex particles of the present invention, the content of the particle size adjusting composition is naturally 70 w% or less. The content of the particle size adjusting composition is calculated according to Formula (4) using the content of the metal complex according to Formulae (1) to (3) described above.

$$\text{Content of particle size adjusting composition (w\%)} = 100 - \text{metal complex content (w\%)} \qquad \text{Formula (4)}$$

As shown in Figure 2A, in the conventional magnetic particles disclosed in Japanese Patent Laid-Open No. 10-55911, a magnetic material ($Fe_3O_4$) has been present in a surface layer part in a cross-sectional view. In addition, in the conventional magnetic particles disclosed in Japanese Patent Application No. 2010-528640 (Japanese Patent No. 5574492), a magnetic material ($Fe_3O_4$) has been locally present as magnetic particles (islands) on a resin (sea) around the core of a particle as shown in Figure 2B, and a fluorescent dye (rare earth (Eu)) has been present in a particle surface layer part. However, in the metal complex particles of the present invention, since particles are formed from the metal complex itself as shown in Figure 1A and Figure 1B, approximately uniform morphology is formed from a surface layer (surface) to the center (point) of a particle in a cross-sectional view of the metal complex particles. That is, as described above, high magnetic collectiveness is achieved by virtue of the high content of the metal complex, and at the same time, since a quantity of magnetism per particle becomes uniform, responses to magnetic force of individual particles become uniform at separation using magnetic force. Therefore, variations during separation become small. The particle size adjusting composition may be locally distributed in the metal complex particles. In such a case, however, a configuration in which the particle size adjusting composition (island) is localized in or dots with the metal complex (sea) is formed. Such a configuration is also one aspect of the present invention.

While the hydrophobic solvent used in the above-described production methods (1), (2), and (3) is not particularly limited, it is preferable that the hydrophobic solvent has an SP value of 10 or less and has a boiling point lower than that of water in order to distill the hydrophobic solvent away afterward. Examples thereof include ethyl acetate (SP value: 9.1, boiling point: 77.1°C), benzene (SP value: 9.2, boiling point: 80.1°C), diisopropyl ether (SP value: 6.9, boiling point: 69°C), and chloroform (SP value: 9.1, boiling point: 61.2°C). These hydrophobic solvents may be used singly, or two or more thereof may be used in combination. Among them, ethyl acetate is preferable.

The SP value means a solubility parameter $\delta$ calculated according to the following Formula (A) using $\Delta F$ and $\Delta v$ values of various atomic groups provided by Nakatsu and described in "Secchaku (Adhesion)," Koubunshikankoukai (polymer publication society), vol. 40, no. 8 (1996), pp. 342-350 by Toshinao Nakatsu. In addition, in the cases of a mixture and a copolymer, the SP value means a solubility parameter $\delta_{mix}$ calculated according to the following Formula (B).

$$\delta = \Sigma\Delta F/\Sigma\Delta v \qquad (A)$$

$$\delta_{mix} = \phi_1\delta_1 + \phi_2\delta_2 + \ldots \phi_n\delta_n \qquad (B)$$

In the formulae, $\Delta F$ and $\Delta v$ represent $\Delta F$ and a molar volume $\Delta v$ of each atomic group provided by Nakatsu, respectively. A volume fraction or a molar fraction is represented by $\phi$, provided that $\phi_1 + \phi_2 + \ldots \phi_n = 1$.

The hydrophobic polymerization initiator used in the above-described production methods (1), (2), and (3) is not particularly limited as long as it is a hydrophobic polymerization initiator capable of dissolving in the above-described hydrophobic solvent. Specific examples thereof include peroxides such as di-tert-butyl peroxide, tert-butyl hydroperoxide, benzoyl peroxide, methyl ethyl ketone peroxide, and di(secondary-butyl) peroxydicarbonate and azo-based compounds such as azobisisobutyronitrile, 2,2'-azobis(2-methylbutyronitrile) (V-59), 2,2'-azobis(2,4-dimethylvaloronitrile) (V-65), 2,2'-azobis(4-methoxy-2,4-dimethylvaloronitrile) (V-70), and dimethyl 2,2'-azobis(isobutyrate) (V-601). However, tert-butyl-2-ethylperoxyhexanoate, di(secondary-butyl) peroxydicarbonate, and azobisisobutyronitrile are preferable.

Examples of the dispersant used in the above-described production methods (1), (2), and (3) include water-soluble polymers such as a polyalkylene glycol, a polyvinyl alcohol, a polyvinyl pyrrolidone, a poly(meth)acrylic acid, a poly(meth)acrylic acid having a water-soluble group such as hydroxy group, carboxy group, a polyalkylene glycol group, or the like in a side chain, a poly(meth)acrylate, colloidal silica, and calcium carbonate. These dispersants may be used singly, or two or more thereof may be used in combination.

The surfactant used in the above-described production methods (1), (2), and (3) is not particularly limited as long as it is water-soluble, and examples thereof include nonionic surfactants such as a polyalkylene glycol alkyl ether; anionic surfactants such as an alkyl sulfate, an alkylbenzene sulfonate, an alkyl carboxylate, an alkyl phosphoric ester salt, and an alkyl sulfuric acid ester salt; cationic surfactants such as an alkyl amine salt, a quaternary alkyl ammonium salt; and amphoteric surfactants such as betaine type, sulfobetaine type, and alkylbetaine type amphoteric surfactants. These surfactants may be used singly, or two or more thereof may be used in combination.

Other additives may be used in the above-described production methods (1), (2), and (3) as needed.

Examples of the other additives include potassium chloride, sodium chloride, sodium acetate, ammonium acetate, sodium citrate, potassium sulfate, sodium sulfate, sodium dihydrogen phosphate, and disodium hydrogen phosphate as an electrolyte. These additives may be used singly, or two or more thereof may be used in combination.

An example of one aspect of the production method of the metal complex particles include a production method of metal complex particles, the method including:

a step of preparing (a) an aqueous medium in which a surfactant or a water-soluble polymer is dissolved;
a step of preparing (A) an aqueous medium dispersion liquid in which particles (template particles) prepared from a particle size adjusting composition are dispersed in the above-described aqueous medium (a);
a step of preparing (b) a hydrophobic solvent capable of dissolving the particle size adjusting composition;
a step of preparing (B) a metal complex solution in which a metal complex having both magnetism and fluorescence is dissolved in the above-described hydrophobic solvent (b);
a step of mixing (A) and (B) described above to prepare (c) a swollen droplet constituted of the particle size adjusting composition, the metal complex, and the hydrophobic solvent (b); and
a step of removing the hydrophobic solvent (b) from the above-described swollen droplet (c).

The metal complex is preferably constituted of holmium ion, terbium ion, or dysprosium ion and both of or at least one of a β-diketone ligand and a heterocyclic ligand.

The hydrophobic polymer preferably includes a styrene-based or (meth)acrylate-based hydrophobic polymer.

Sensitized metal complex particles can be prepared by immobilizing a measurement object substance, an analog of a measurement object substance, or a substance specifically binding to a measurement object substance (hereinafter these substances are sometimes collectively abbreviated as a substance for carrying), with the metal complex particles having both fluorescence and magnetism according to the present invention being a carrier. These sensitized metal complex particles are also one aspect of the present invention.

The measurement object substance in the present invention is not limited as long as it is usually measured in this field, and representative examples thereof include proteins, lipid proteins, nucleic acids, immune globulins, blood coagulation-related factors, antibodies, enzymes, hormones, cancer markers, cardiac disease markers, various medicines, and the like contained in a sample originating from a living body such as biological fluids such as blood serum, blood, blood plasma, and urine, lymph fluids, blood cells, a variety of cells, and the like. More specifically, examples thereof include proteins such as albumin, hemoglobin, myoglobin, transferrin, and C-reactive protein (CRP); lipoproteins such as high-density lipoprotein (HDL), low-density lipoprotein (LDL), and very low-density lipoprotein; nucleic acids such as deoxyribonucleic acid (DNA) and ribonucleic acid (RNA); enzymes such as alkali transferase, amylase, acid phosphatase, γ-glutamyl transferase (γ-GTP), lipase, creatinine kinase (CK), lactate dehydrogenase (LDH), glutamic oxaloacetic transaminase (GOT), glutamic pyruvic transaminase (GPT), renin, protein kinase (PK), and tyrosine kinase; immune globulins such as IgG, IgM, IgA, IgD, and IgE (or fragments such as Fc part, Fab part, and $F(ab)_2$ part thereof); blood coagulation-related factors such as fibrinogen, a fibrin degradation products (FDPs), prothrombin, and thrombin; antibodies such as an anti-streptolysin O antibody, an anti-human hepatitis B virus surface antigen antibody (HBs antigen), an anti-human hepatitis C virus antibody, and an antirheumatoid factor; hormones such as thyroid stimulating hormone (TSH), thyroid hormones (FT3, FT4, T3, and T4), parathyroid hormone (PHT), human chorionic gonadotropin (hCG), and estradiol (E2); cancer markers such as α-fetoprotein (AFP), carcinoembryonic antigens (CEAs), CA19-9, and prostate specific antigen (PSA); cardiac disease markers such as troponin T (TnT) and N-terminal fragment of human pro-brain natriuretic peptide (NT-proBNP); and medicines such as an antiepilepsy drug, an antibiotic, and theophylline.

An analog (analog) of a measurement object substance in the present invention may be any substance which may bind to a binding site which a substance (measurement object substance-binding substance) specifically binding to the measurement object substance has and which binds to the measurement object substance, in other words, any substance having a binding site which the measurement object substance has and which binds to the measurement object substance-binding substance, further in other words, any substance which may compete with a reaction between the measurement object substance and the measurement object substance-binding substance when the substance coexists during the reaction.

Examples of the substance (measurement object substance-binding substance) specifically binding to a measurement object substance in the present invention include a substance binding to a measurement object substance or an analog thereof through a mutual reaction such as an "antigen"-"antibody" reaction, a "sugar chain"-"protein" reaction, a "sugar chain"-"lectin" reaction, an "enzyme"-"inhibitor" reaction, a "protein"-"peptide chain" reaction, a "chromosome or nucleotide chain"-"nucleotide chain" reaction, or a "nucleotide chain"-"protein" reaction. When one reactant of the above-described combinations is a measurement object substance or an analog thereof, the other one is the measurement object substance-binding substance. For example, when a measurement object substance or an analog thereof is an "antigen," the measurement object substance-binding substance is an "antibody," and when a measurement object

substance or an analog thereof is an "antibody," the measurement object substance-binding substance is an "antigen" (the same is applied to the following other combinations described above).

Specifically, examples thereof include nucleotide chains (an oligonucleotide chain or a polynucleotide chain); chromosomes; peptide chains (for example, C-peptide, angiotensin, etc.); proteins [for example, procalcitonin, immunoglobulin A (IgA), immunoglobulin E (IgE), immunoglobulin G (IgG), immunoglobulin M (IgM), immunoglobulin D (IgD), β2-microvlobulin, albumin, a degradation product thereof, and a serum protein such as ferritin); enzymes [for example, an amylase (for example, pancreatic-type, salivary-type, X-type, etc.), alkaline phosphatase (for example, hepatic, bony, placental, small-intestinal, etc.), acid phosphatase (for example, PAP, etc.), γ-glutamyl transferase (for example, renal, pancreatic, hepatic, etc.), lipase (for example, pancreatic-type, gastric-type, etc.), creatine kinase (for example, CK-1, CK-2, mCK, etc.), lactate dehydrogenase (for example, LDH1 to LDH 5, etc.), glutamic oxaloacetic transaminase (for example, ASTm, ASTs, etc.), glutamic pyruvic transaminase (for example, ALTm, ALTs, etc.), cholinesterase (for example, ChE1 to ChE5, etc.), leucine aminopeptidase (for example, C-LAP, AA, CAP, etc.), renin, protein kinase, tyrosine kinase, and the like] and inhibitors of these enzymes; hormones (for example, PTH, TSH, insulin, LH, FSH, prolactin, etc.); receptors (for example, a receptor to estrogen, TSH, or the like); ligands (for example, estrogen, TSH, etc.); microorganisms such as bacteria (for example, *Mycobacterium tuberculosis, Streptococcus pneumoniae, Corynebacterium diphtheriae, Neisseria meningitidis, Neisseria gonorrhoeae, Staphylococcus* species, *Streptococcus* species, intestinal bacteria, *Escherichia coli, Helicobacter pylori,* etc.), viruses (for example, rubella virus, herpes viruses, hepatitis viruses, ATL virus, AIDS virus, influenza viruses, adenoviruses, enteroviruses, poliovirus, EB virus, HAV, HBV, HCV, HIV, HTLV, etc.), fungi (for example, *Candida* species, *Cryptococcus* species, etc.), spirochetes (for example, *Leptospira* species, *Treponema pallidum,* etc.), chlamydias, mycoplasmas, and the like; a protein or a peptide or a carbohydrate antigen originating from said microorganisms; various allergens causing allergies such as bronchial asthma, allergic rhinitis, and atopic dermatitis (for example, allergens originating from house dust, mites such as *Dermatophagoides farinae, Dermatophagoides pteronyssinus,* pollen dust of, for exemple, *Cryptomeria japonica, Chamaecyparis obtusa, Paspalum thunbergii* Kunth ex Steud., *Ambrosia artemisiifolia, Phleum pretense, Anthoxanthum odoratum, Secale cereale,* etc., animals such as cats, dogs, and crabs, foods such as rice and egg white, fungi, insects, woods, medicines, chemicals, and the like); lipids (for example, lipoprotein, etc.); proteases (for example, trypsin, plasmin, serine protease, etc.); tumor marker protein antigens (for example, PSA, PGI, PGII, etc.); sugar chain antigens [for example, AFP (for example, L1 to L3, etc.), hCG (hCG family), transferrin, IgG, thyroglobulin, Decay-accelerating-factor (DAF), carcinoembryonic antigens (for example, CEA, NCA, NCA-2, NFA, etc.), CA19-9, PIVKA-II, CA125, prostate specific antigen, a tumor marker sugar chain antigen having a specific sugar chain produced by a cancer cell, ABO sugar chain antigens, etc.]; sugar chains (for example, hyaluronic acid, β-glucan, a sugar chain contained in the above-described antigens and the like, etc.); a protein bonding to a sugar chain (for example, a hyaluronic acid-binding protein, a β-glucan-binding protein, etc.); phospholipids (for example, cardiolipin, etc.); lipopolysaccharides (for example, endotoxin, etc.); chemicals (for example, T3, T4, and endocrine-disrupting chemicals such as tributyltin, nonylphenol, 4-octylphenol, di-n-butyl phthalate, dichlorohexyl phthalate, benzophenone, octachlorostylene, and di-2-ethylhexyl phthalate); various medicines administered and inoculated into a human body and metabolites thereof; aptamers; nucleic acid-binding substances; antigens against them; and the like.

Degradation products such as Fab and $F(ab')_2$ fragments produced by a protein-degrading enzyme such as papain and pepsin or by chemical degradation are included in the antibodies used for the present invention in addition to immune globulin molecules per se. In addition, either of a polyclonal antibody or a monoclonal antibody may be used for the above-described antibodies. A commonly used method can be used as a method for obtaining the above-described antibodies.

The measurement object substance-binding substance as described above is preferably a substance binding to a measurement object substance or an analog thereof through an "antigen"-"antibody" reaction or a "sugar chain-protein" reaction. Specifically, an antibody against a measurement object substance or an analog thereof, an antigen to which a measurement object substance or an analog thereof binds, or a protein binding to a measurement object substance or an analog thereof is preferable, and an antibody against a measurement object substance or an analog thereof or an antigen binding to a measurement object substance or an analog thereof is more preferable. The terms "antigen-antibody reaction," "antigen," and "antibody" used in the present description may mean to include both of the above-described concept and forms in which the sensitized metal complex particles are aggregated by a specific binding reaction in addition to the usual meaning and should not be interrupted in a limited manner.

A method for immobilizing a substance for carrying on the metal complex particles of the present invention to produce sensitized metal complex particles is not particularly limited. A conventionally known method for immobilization through physical and/or chemical bonding can be used. Examples of a chemically bonding method include a method for introducing at least one organic compound selected from the group consisting of aldehyde, albumin, carbodiimide, streptavidin, biotin, and epoxide and/or at least one functional group selected from amino group, carboxy group, hydroxy group, mercapto group, glycidyloxy group, tosyl group, and the like into metal complex particle surfaces, and immobilizing the substance for carrying on the metal complex particles therethrough. Among them, an organic compound containing

a carboxy group or a tosyl group is especially preferable in view of stability of the metal complex particles themselves and immobilization efficiency of the substance for carrying.

The binding amount of the substance for carrying in the sensitized metal complex particles of the present invention varies according to the kind of the substance for carrying used, and an experimentally optimal amount can be appropriately set. In the present description, the terms "carry," "sensitize," and "immobilize" have respective usual meanings and are used in the same senses.

The sensitized metal complex particles of the present invention obtained by such a method are subjected to coating (blocking) treatment with a variety of polymer compounds or proteins (for example, bovine serum albumin, etc.) as needed, dispersed in an appropriate buffer solution, and used as a sensitized metal complex particle dispersion liquid. The sensitized metal complex particle dispersion liquid can be used as an immunoassay reagent and said assay reagent is also one aspect of the present invention. The immunoassay reagent of the present invention can be combined with a diluent (buffer solution), a standard substance, and the like used for measurements and used as an assay reagent kit, and said kit is also one aspect of the present invention.

The above-described diluent is used for diluting a sample to be measured and the like. Any buffer solution having pH of 5.0 to 9.0 can be used as the above-described diluent. Specifically, examples thereof include a phosphate buffer solution, a glycine buffer solution, a tris buffer solution, a boric acid buffer solution, a citric acid buffer solution, and a Good's buffer solution.

The immunoassay reagent and the diluent of the present invention may contain a variety of sensitizers for the purposes of improving measurement sensitivity and accelerating the specific reaction between a measurement object substance and a substance for carrying. Examples of the above-described sensitizer include alkylated polysaccharides such as methyl cellulose and ethyl cellulose, pullulan, polyvinylpyrrolidone, and the like.

The immunoassay reagent and the diluent of the present invention may contain a variety of polymer compounds and proteins and a degradation product thereof, an amino acid, a surfactant, and the like for the purposes of suppressing nonspecific reactions caused by a substance other than a measurement object substance present in a measurement sample, improving stability of the assay reagent, and the like. Examples of such additives include proteins such as albumin (bovine serum albumin, ovalbumin), casein, and gelatin.

The method for measuring a measurement object substance according to the present invention is not particularly limited as long as the method uses the metal complex particles of the present invention described above and may be conducted according to sandwich method, competitive method, and the like usually conducted in this field and described in literatures (for example, "Kousomenekisokuteihou (Enzyme immunoassay), second edition," edited by Eiji Ishikawa et al., IGAKU-SHOIN Ltd., 1982), for example.

In the method for measuring a measurement object substance according to the present invention, a method for allowing a sample, metal complex particles, a labeled measurement object substance-binding substance, a measurement object substance or an analog thereof, and the like to contact with each other may be any method in which the metal complex particles are dispersed through regular treatment such as stirring and mixing. A reaction time may be appropriately set according to a measurement object substance, a measurement object substance-binding substance to be used, the difference between sandwich method and competitive method, and the like.

B/F separation (separation of binding labeling antibodies and non-binding labeling antibodies) in the method for measuring a measurement object substance according to the present invention is conducted by collecting metal complex particles by a magnet or the like from the outside of a reaction vessel and the like utilizing magnetism of the metal complex particles, discharging the reaction solution, adding a washing liquid, subsequently removing the magnet, and mixing the metal complex particles to disperse the metal complex particles followed by washing, for example. The above-mentioned operations may be repeated one to three times. The washing liquid is not particularly limited as long as it is usually used in this field.

Examples of a labeling substance used for labeling a measurement object substance-binding substance and a measurement object substance or an analog thereof include enzymes such as alkaline phosphatase, β-galactosidase, peroxidase, microperoxidase, glucose oxidase, glucose-6-phosphate dehydrogenase, malate dehydrogenase, luciferase, tyrosinase, and acid phosphatase used in enzyme immunoassay (EIA), for example; radioactive isotopes such as $^{99}$mTc, $^{131}$I, $^{125}$I, $^{14}$C, $^{3}$H, and $^{32}$P used in radioimmunoassay (RIA), for example; fluorescein, dansyl, fluorescamine, coumarin, and naphthylamine or derivatives thereof used in fluoroimmunoassay (FIA), for example; fluorescent substances such as green fluorescent protein (GFP); light-emitting substances such as luciferin, isoluminol, luminol, bis-(2,4,6-trichlorophenyl)oxalate; substances showing absorption in an ultraviolet region such as phenol, naphthol, and anthracene or derivatives thereof; and substances having characteristics as a spin-labeling agent represented by a compound having an oxyl group such as 4-amino-2,2,6,6-tetramethylpyperidin-1-oxyl, 3-amino-2,2,5,5-tetramethylpyrrolidin-1-oxyl, and 2,6-dit-butyl-α-(3,5-di-t-butyl-4-oxo-2,5-cyclohexadien-1-ylidene)-p-trioxyl. Among them, enzymes and fluorescent substances are preferable, alkaline phosphatase, peroxidase, and glucose oxidase are more preferable, and peroxidase is especially preferable in view of sensitivity.

A method usually used in this field, for example, a labeling method known per se and commonly conducted in

EIA, RIA, FIA, or the like known per se [for example, the glutaraldehyde method, periodic acid method, maleimide method, pyridyldisulfide method, or the like described in Ikagakujikkenkouza (Medical chemistry experiment course), volume 8, supervised by Yuichi Yamamura, first edition, Nakayama Shoten Co., Ltd., 1971; Zusetsu keikoukoutai (Illustrated fluorescent antibodies), written by Akira Kawaoi, first edition, Soft Science Co., Ltd.,1983; Kousomenekisokuteihou (Enzyme immunoassay), edited by Eiji Ishikawa, Tadashi Kawai, and Kiyoshi Muroi, second edition, IGAKU-SHOIN Ltd., 1982), etc.] or the like may be used to bind the labeling material described above to a measurement object substance-binding substance and a measurement object substance or an analog thereof

An amount of the labeling substance to be used may be appropriately set according to the kind of the labeling substance to be used. For example, the labeling substance may be contained in a buffer solution usually used in this field such as a tris buffer solution, a phosphate buffer solution, a veronal buffer solution, a boric acid buffer solution, and a Good's buffer solution. Examples of the buffer solution include a tris buffer solution, a phosphate buffer solution, a veronal buffer solution, a boric acid buffer solution, and a Good's buffer solution usually used in this field. The pH value of the buffer solution may be within a range not inhibiting antigen-antibody reactions and is usually 5 to 9. In addition, a stabilizer such as albumin, globulin, water-soluble gelatin, and polyethylene glycol, a surfactant, a sugar, and the like may be added to such buffer solutions in advance as long as they do not inhibit a target antigen-antibody reaction.

A usually conducted method can be used as a method for measuring the labeling substance or activity thereof with no specific limitation. An optical device used for measurements is also not particularly limited. Representatively, any automatic biochemical analyzer widely used for clinical assays can be used.

## Examples

Hereinafter, the present invention will be described with reference to Examples. However, the present invention is not interpreted as being limited to the following Examples.

[Preparation Example 1]

Synthesis of metal complex (a)

To an ethanol solution of holmium chloride hexahydrate ($HoCl_3 \cdot 6H_2O$), 2,2'-bipyridyl (bpy) and dibenzoylmethane (DBM) were added in an amount of one equivalent and an amount of three equivalents based on holmium chloride hexahydrate, respectively. After mixing the resultant mixture, diethanolamine was further added thereto to adjust its pH to 6-7. After stirring the resultant mixture for 24 hours at room temperature, a pale-yellow precipitate was obtained. The obtained precipitate was filtered and subsequently washed with ethanol to obtain a metal complex (a) whose metal ion was holmium and whose ligands were bpy and DBM.

[Preparation Example 2]

Synthesis of metal complex (b)

Operations similar to those of Preparation Example 1 were conducted except that thenoyl trifluoroacetone (TTA) was used as a ligand instead of DBM to obtain a metal complex (b) whose metal ion was holmium and whose ligands were bpy and TTA.

[Preparation Example 3]

Synthesis of metal complex (c)

Operations similar to those of Preparation Example 1 were conducted except that dysprosium chloride ($DyCl_3$) was used instead of holmium chloride hexahydrate to obtain a metal complex (c) whose metal ion was dysprosium and whose ligands were bpy and DBM.

[Preparation Example 4]

Synthesis of metal complex (d)

Operations similar to those of Preparation Example 1 were conducted except that terbium chloride hexahydrate ($TbCl_3 \cdot 6H_2O$) was used instead of holmium chloride hexahydrate to obtain a metal complex (d) whose metal ion was terbium and whose ligands were bpy and DBM.

[Preparation Example 5]

Synthesis of metal complex (e)

Operations similar to those of Preparation Example 1 were conducted except that 2,2':6,2''-terpyridine (terpy) was used as a ligand instead of bpy to obtain a metal complex (e) whose metal ion was holmium and whose ligands were terpy and DBM.

[Preparation Example 6]

Synthesis of metal complex (f)

Operations similar to those of Preparation Example 1 were conducted except that 1,10-phenanthroline monohydrate (phen) was used as a ligand instead of bpy to obtain a metal complex (f) whose metal ion was holmium and whose ligands were phen and DBM.

[Preparation Example 7]

Synthesis of metal complex (g)

Operations similar to those of Preparation Example 1 were conducted except that europium chloride hexahydrate ($EuCl_3 \cdot 6H_2O$) was used instead of holmium chloride hexahydrate and that thenoyltrifluoroacetone (TTA) was used as a ligand to obtain a metal complex (g) whose metal ion was europium and whose ligand was TTA.

[Preparation Example 8]

Preparation of template particles (I)

To a reaction vessel, 500 g of ion-exchanged water, 50 g of methyl methacrylate (MMA) (manufactured by Wako Pure Chemical Industries, Ltd.) as a monomer constituting the particle size adjusting composition, and 0.5 g of potassium persulfate (manufactured by Wako Pure Chemical Industries, Ltd.) were added, and the resultant mixture was stirred at room temperature for two hours under a nitrogen atmosphere. The resultant mixture was subsequently heated to 70°C and polymerization was conducted for 24 hours to obtain template particles (I) including polymethyl methacrylate.

The average particle size and CV value of the obtained template particles (I) were 1.06 μm and 3.3%, respectively.

[Preparation Example 9]

Preparation of template particles (II)

Template particles (II) including polystyrene were obtained by similar operations except that the monomer constituting the particle size adjusting composition was changed from MMA to styrene (St) (manufactured by NS Styrene Monomer Co., Ltd.).

The average particle size and CV value of the obtained template particles (II) were 1.03 μm and 3.8%, respectively.

[Example 1]

Preparation of metal complex particles (1)

The above-described template particles (I) and triethanolamine lauryl sulfate (Emal TD, manufactured by Kao Corporation) were added to ion-exchanged water so that the content of the above-described template particles (I) became 0.1 g and the content of triethanolamine lauryl sulfate became 0.1 g in 100 g of ion-exchanged water to obtain a template particle (I) dispersion liquid. Then, a metal complex (a) solution prepared by dissolving 2.70 g of the metal complex (a) in 4.00 g of ethyl acetate was added to the above-described template particle (I) dispersion liquid. A complex droplet of the above-described template particles (I) and the metal complex (a) was formed through agitation at room temperature for 24 hours. Agitation was subsequently performed for ten hours while heating at 80°C, and ethyl acetate was removed

to obtain metal complex particles (1) of the present invention.

The average particle size and CV value of the obtained metal complex particles (1) were 3.21 μm and 3.8%, respectively, and the metal complex content thereof was 96.4 w%.

[Example 2]

The above-described template particles (I) and triethanolamine lauryl sulfate (Emal TD, manufactured by Kao Corporation) were added to ion-exchanged water so that the content of the above-described template particles (I) became 0.1 g and the content of triethanolamine lauryl sulfate became 0.1 g in 100 g of ion-exchanged water to obtain a template particle (I) dispersion liquid. Then, a metal complex (a) solution prepared by dissolving 2.50 g of the metal complex (a), 0.20 g of methyl methacrylate (manufactured by Wako Pure Chemical Industries, Ltd.) as a monomer for the second particle size adjusting composition, and 0.02 g of di(secondary-butyl) peroxydicarbonate (Luperox 225, manufactured by ARKEMA Yoshitomi, Ltd.) in 4.00 g of ethyl acetate was added to the above-described template particle (I) dispersion liquid. A complex droplet of the above-described template particles (I) and the metal complex (a) was formed through agitation at room temperature for 24 hours. Agitation was subsequently performed for ten hours while heating at 80°C, and methyl methacrylate was polymerized while removing ethyl acetate to obtain metal complex particles (2) containing polymethyl methacrylate (pMMA) as the second particle size adjusting composition.

The average particle size and CV value of the obtained metal complex particles (2) were 3.20 μm and 4.2%, respectively, and the metal complex content thereof was 89.1 w%.

[Example 3]

Metal complex particles (3) containing polydivinylbenzene (pDVB) as the second particle size adjusting composition were obtained by operations similar to those of Example 2 except that divinylbenzene (DVB) (DVB960, manufactured by NS Styrene Monomer Co.,Ltd.) was used as a monomer for the second particle size adjusting composition.

The average particle size and CV value of the obtained metal complex particles (3) were 3.18 μm and 4.1%, respectively, and the metal complex content thereof was 88.1 w%.

[Example 4]

Metal complex particles (4) containing polystyrene (pSt) as the second particle size adjusting composition were obtained by operations similar to those of Example 2 except that the template particles (II) were used and styrene (St) (manufactured by NS Styrene Monomer Co.,Ltd.) was used as a monomer for the second particle size adjusting composition.

The average particle size and CV value of the obtained metal complex particles (4) were 3.10 μm and 4.3%, respectively, and the metal complex content thereof was 89.0 w%.

[Example 5]

Metal complex particles (5) were obtained by operations similar to those of Example 3 except that the metal complex (b) was used as the metal complex.

The average particle size and CV value of the obtained metal complex particles (5) were 3.12 μm and 4.2%, respectively, and the metal complex content thereof was 88.2 w%.

[Example 6]

Metal complex particles (6) were obtained by operations similar to those of Example 3 except that 2.00 g of the metal complex (a), 0.70 g of divinylbenzene, and 0.08 g of di(secondary-butyl) peroxydicarbonate were used.

The average particle size and CV value of the obtained metal complex particles (6) were 3.20 μm and 3.8%, respectively, and the metal complex content thereof was 71.4 w%.

[Example 7]

Metal complex particles (7) were obtained by operations similar to those of Example 3 except that 1.60 g of the metal complex (a), 1.10 g of divinylbenzene, and 0.11 g of di(secondary-butyl) peroxydicarbonate were used.

The average particle size and CV value of the obtained metal complex particles (7) were 3.18 μm and 3.9%, respectively, and the metal complex content thereof was 55.6 w%.

[Example 8]

Metal complex particles (8) were obtained by operations similar to those of Example 3 except that 1.20 g of the metal complex (a), 1.50 g of divinylbenzene, and 0.15 g of di(secondary-butyl) peroxydicarbonate were used.
The average particle size and CV value of the obtained metal complex particles (8) were 3.17 μm and 4.1%, respectively, and the metal complex content thereof was 40.2 w%.

[Example 9]

A mixture obtained by mixing 2.50 g of the metal complex (a), 0.20 g of styrene, 13 g of chloroform, 0.002 g of di(secondary-butyl) peroxydicarbonate, 13 g of 5% by weight aqueous solution of polyvinyl alcohol (GOHSENOL GH20, manufactured by Nippon Synthetic Chemical Industry Co.,Ltd.), 1.5 g of triethanolamine lauryl sulfate, and 150 g of ion-exchanged water was stirred at 2500 rpm using a homogenizer. The resultant mixture was subsequently transferred to a reaction vessel, stirred at 200 rpm for two hours under a flow of nitrogen, and subsequently stirred at 80°C for ten hours to polymerize styrene and remove chloroform at the same time. After the completion of the reaction, centrifugation operations were repeated to obtain metal complex particles (9) containing polystyrene (pSt) as the particle size adjusting composition.
The average particle size and CV value of the obtained metal complex particles (9) were 3.31 μm and 12.8%, respectively, and the metal complex content thereof was 90.2 w%.

[Example 10]

Metal complex particles (10) of to the present invention were obtained by operations similar to those of Example 9 except that centrifugation operations were repeated with centrifugation conditions in the centrifugation operations after the completion of the reaction in Example 9 changed.
The average particle size and CV value of the obtained metal complex particles (10) were 3.42 μm and 17.8%, respectively, and the metal complex content thereof was 91.3 w%.

[Example 11]

Metal complex particles (11) were obtained by operations similar to those of Example 3 except that the metal complex (c) was used as the metal complex.
The average particle size and CV value of the obtained metal complex particles (11) were 3.22 μm and 4.8%, respectively, and the metal complex content thereof was 89.3 w%.

[Example 12]

Metal complex particles (12) were obtained by operations similar to those of Example 3 except that the metal complex (d) was used as the metal complex.
The average particle size and CV value of the obtained metal complex particles (12) were 3.16 μm and 4.4%, respectively, and the metal complex content thereof was 88.7 w%.

[Example 13]

Metal complex particles (13) were obtained by operations similar to those of Example 3 except that the metal complex (e) was used as the metal complex.
The average particle size and CV value of the obtained metal complex particles (13) were 3.22 μm and 5.2%, respectively, and the metal complex content thereof was 89.2 w%.

[Example 14]

Metal complex particles (14) were obtained by operations similar to those of Example 3 except that the metal complex (f) was used as the metal complex.
The average particle size and CV value of the obtained metal complex particles (14) were 3.06 μm and 4.9%, respectively, and the metal complex content thereof was 87.5 w%.

[Comparative Example 1]

Metal complex particles (15) were obtained by operations similar to those of Example 3 except that the amount of charge of the metal complex (a) was changed to 0.50 g and the amount of charge of divinylbenzene as the second particle size adjusting composition was changed to 2.20 g.
The average particle size and CV value of the obtained metal complex particles (15) were 3.18 μm and 5.1%, respectively, and the metal complex content thereof was 14.8 w%.

[Comparative Example 2]

Metal complex particles (16) of to the present invention were obtained by operations similar to those of Example 9 except that centrifugation operations were repeated with centrifugation conditions in the centrifugation operations after the completion of the reaction in Example 9 changed.
The average particle size and CV value of the obtained metal complex particles (16) were 3.33 μm and 25.6%, respectively, and the metal complex content thereof was 90.4 w%.

[Comparative Example 3]

Metal complex particles (17) were obtained by operations similar to those of Example 3 except that the metal complex (g) was used as the metal complex.
The average particle size and CV value of the obtained metal complex particles (17) were 3.15 μm and 4.8%, respectively, and the metal complex content thereof was 88.6 w%.

[Evaluation method]

* Evaluation on magnetic collectiveness

In a state where a magnet (3680 G, W 10 mm × D 10 mm × H 5 mm) was applied to a spectrophotometer (U-3900H, manufactured by Hitachi, Ltd.) in advance with a spacer (W 10 mm × D 10 mm × H 10 mm) interposed therebetween, a sample (1.2 mL) was input into a quartz cell placed in the spectrophotometer, absorbance was measured from 5 seconds to 125 seconds after the sample input, and a magnetism collection rate was calculated using Formula (6) .

Magnetism collection rate (%) = [{(absorbance 5 seconds after sample input) - (absorbance 125 seconds after sample input)}/(absorbance 5 seconds after sample input)] × 100     Formula (6)

[Determination criteria]

Excellent: magnetism collection rate is 61% or more
Good: magnetism collection rate is 41% or more and less than 60%
Fair: magnetism collection rate is 21% or more and less than 40%
Poor: magnetism collection rate is less than 20%
Obtained results are collectively shown in Table 1, Table 2, and Table 3.

[Reference Example 1]

In the production method of Patent Literature 3 (Japanese Patent Laid-Open No. 2008-127454), a magnetic material ($Fe_3O_4$) is locally present around the core of a particle and a fluorescent dye (rare earth (Eu)) is present in a particle surface layer part as shown in Figure 2B. Therefore, the color of the particle is brown to black. In addition, the particle size is about 0.2 μm and the ferrite particle content is about 5 w%. The 120s magnetic collectiveness was 8%, and the relative fluorescence intensity was 36.

[Reference Example 2]

In the production method described in Japanese Patent Laid-Open No. 10-55911, a magnetic material ($Fe_3O_4$) is present in a surface layer part of a particle as shown in Figure 2A. In addition, since no fluorescent dye is contained, no fluorescence is exhibited. The ferrite content was 18.3 w% and the 120s magnetic collectiveness was 83%.

[Reference Example 3]

In the production method described in a reference patent (National Publication of International Patent Application No. 59-500691), a magnetic material ($Fe_3O_4$) is present in the inside of particles. In addition, since no fluorescent dye is contained, no fluorescence is exhibited. The ferrite content was 31.2 w% and the 120s magnetic collectiveness was 95%.

[Table 1]

| | | | Example 1 | Example 2 | Example 3 | Example 4 | Example 5 | Example 6 | Example 7 | Example 8 |
|---|---|---|---|---|---|---|---|---|---|---|
| Particle configuration | Metal complex | Metal | Ho | Ho | Ho | Ho | Ho | Ho | Ho | Ho |
| | | Ligand 1 | bpy | bpy | bpy | bpy | bpy | bpy | bpy | bpy |
| | | Ligand 2 | DBM | DBM | DBM | DBM | TTA | DBM | DBM | DBM |
| | | Content/w% | 96.4 | 89.1 | 88.1 | 89.0 | 88.2 | 71.4 | 55.6 | 40.2 |
| | Particle size adjusting composition | Particle size adjusting composition | pMMA | pMMA | pMMA | pSt | pMMA | pMMA | pMMA | pMMA |
| | | Second particle size adjusting composition | - | pMMA | pDVB | pSt | pDVB | pDVB | pDVB | pDVB |
| | | Total content/w% | 3.6 | 10.9 | 11.9 | 11.0 | 11.8 | 28.6 | 44.4 | 59.8 |
| Physical properties of particles | Particle size/μm | | 3.21 | 3.20 | 3.18 | 3.10 | 3.12 | 3.20 | 3.18 | 3.17 |
| | CV value/% | | 3.8 | 4.2 | 4.1 | 4.3 | 4.2 | 3.8 | 3.9 | 4.1 |
| Numerical values | 120s magnetism collection rate/% | | 70 | 65 | 64 | 65 | 63 | 54 | 44 | 32 |
| | Relative fluorescence intensity/ | | 100 | 98 | 98 | 98 | 97 | 85 | 72 | 52 |
| Evaluation | 120s magnetism collection rate/% | | Excellent | Excellent | Excellent | Excellent | Excellent | Good | Good | Fair |
| | Relative fluorescence intensity/ | | Fair | Fair | Fair | Fair | Fair | Fair | Fair | Fair |

[Table 2]

| | | | Example 9 | Example 10 | Example 11 | Example 12 | Example 13 | Example 14 | Comparative Example 1 | Comparative Example 2 | Comparative Example 3 |
|---|---|---|---|---|---|---|---|---|---|---|---|
| Particle configuration | Metal complex | Metal | Ho | Ho | Dy | Tb | Ho | Ho | Ho | Ho | Eu |
| | | Ligand 1 | bpy | bpy | bpy | bpy | terpy | Phen | bpy | bpy | - |
| | | Ligand 2 | DBM | DBM | DBM | DBM | DBM | DBM | DBM | DBM | TTA |
| | | Content/w% | 90.2 | 91.3 | 89.3 | 88.7 | 89.2 | 87.5 | 148 | 90.4 | 88.6 |
| | Particle size adjusting composition | Particle size adjusting composition | pSt | pSt | pMMA | pMMA | pMMA | pMMA | pMMA | pSt | pMMA |
| | | Second particle size adjusting composition | - | - | pDVB | pDVB | pDVB | pDVB | pDVB | - | pDVB |
| | | Total content/w% | 9.8 | 8.7 | 10.7 | 11.3 | 10.8 | 12.5 | 85.2 | 9.6 | 11.4 |
| Physical properties of particles | Particle size/μm | | 3.31 | 3.42 | 3.22 | 3.16 | 3.22 | 3.06 | 3.18 | 3.33 | 3.15 |
| | CV value/% | | 12.8 | 17.8 | 4.8 | 4.4 | 5.2 | 4.9 | 5.1 | 25.6 | 4.8 |
| Numerical values | 120s magnetism collection rate/ % | | 53 | 40 | 61 | 56 | 64 | 64 | 12 | 19 | 2 |
| | Relative fluorescence intensity/ | | 82 | 71 | 98 | 97 | 98 | 99 | 18 | 74 | 82 |
| Evaluation | 120s magnetism collection rate/ % | | Good | Fair | Excellent | Good | Excellent | Excellent | Poor | Poor | Poor |
| | Relative fluorescence intensity/ | | Fair | Fair | Fair | Fair | Fair | Fair | Poor | Fair | Fair |

[Table 3]

| | Reference Example 1 | Reference Example 2 | Reference Example 3 |
|---|---|---|---|
| 120s magnetism collection rate/% | 8 | 83 | 95 |
| Relative fluorescence intensity/ | 36 | 0 | 0 |

**Claims**

1. Metal complex particle comprising a magnetic material and a particle size adjusting composition containing a hydrophobic polymer, wherein
the magnetic material comprises a metal complex consisting of a metal ion and a ligand and having both magnetism and fluorescence, and
a content of the particle size adjusting composition in the metal complex particle is less than 60 w%.

2. The metal complex particle according to claim 1, wherein the magnetic material is free of a metal complex not having fluorescence but having magnetism.

3. The metal complex particle according to claim 1, wherein the magnetic material consists of the metal complex.

4. The metal complex particle according to any one of claims 1 to 3, wherein a magnetic moment of the metal ion forming the metal complex is 7.5 to 10.7.

5. The metal complex particle according to any one of claims 1 to 4, wherein the metal ion forming the metal complex is at least one selected from the group consisting of ions of terbium, dysprosium, holmium, and erbium.

6. The metal complex particle according to any one of claims 1 to 5, wherein the ligand forming the metal complex is both of or any one of a β-diketone ligand and a heterocyclic ligand.

7. The metal complex particle according to any one of claims 1 to 6, wherein the ligand forming the metal complex is selected from

   (1) dibenzoylmethane or thenoyl fluoroacetone for the β-diketone ligand and
   (2) 2,2'-bipyridyl, 2,2':6,2''-terpyridine, or 1,10-phenanthroline for the heterocyclic ligand.

8. The metal complex particle according to any one of claims 1 to 7, wherein an average particle size of the metal complex particle is 1 μm to 5 μm.

9. The metal complex particle according to any one of claims 1 to 8, wherein a content of the metal complex in the metal complex particle is 40 w% to 98 w%.

10. The metal complex particle according to any one of claims 1 to 9, having a substance for carrying on the surface thereof.

11. The metal complex particle according to any one of claims 1 to 10, wherein the substance for carrying is an antibody or an antigen that specifically reacts with a detection object substance.

12. An immunoassay reagent comprising the metal complex particle according to claim 10.

FIG. 1A

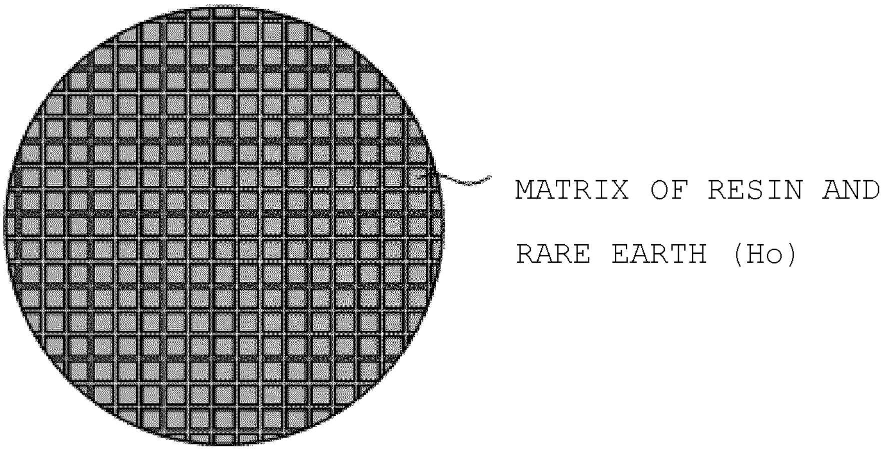


FIG.1B

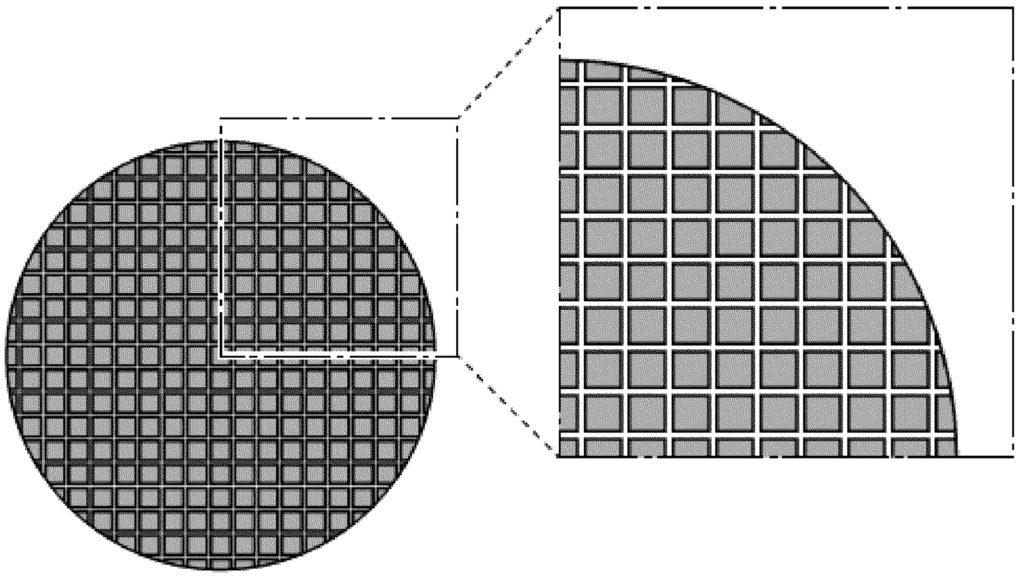

FIG.2A

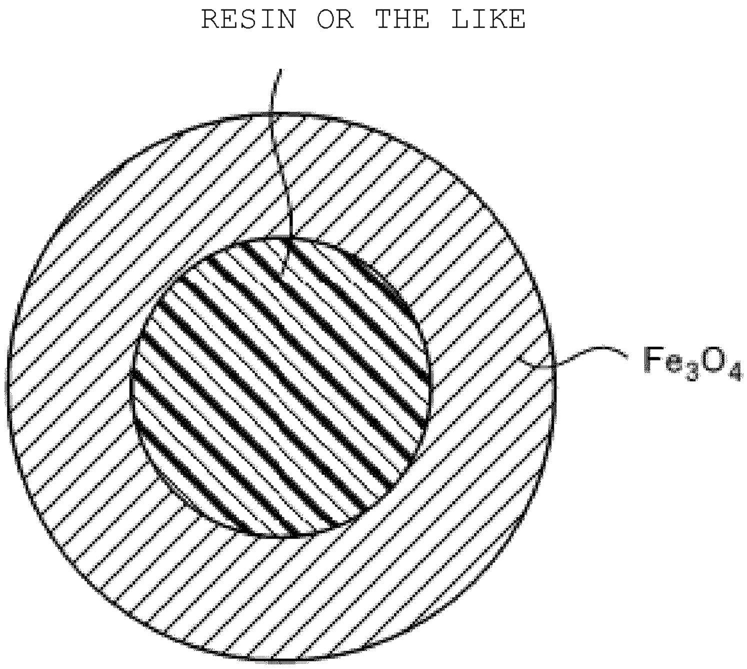


FIG.2B

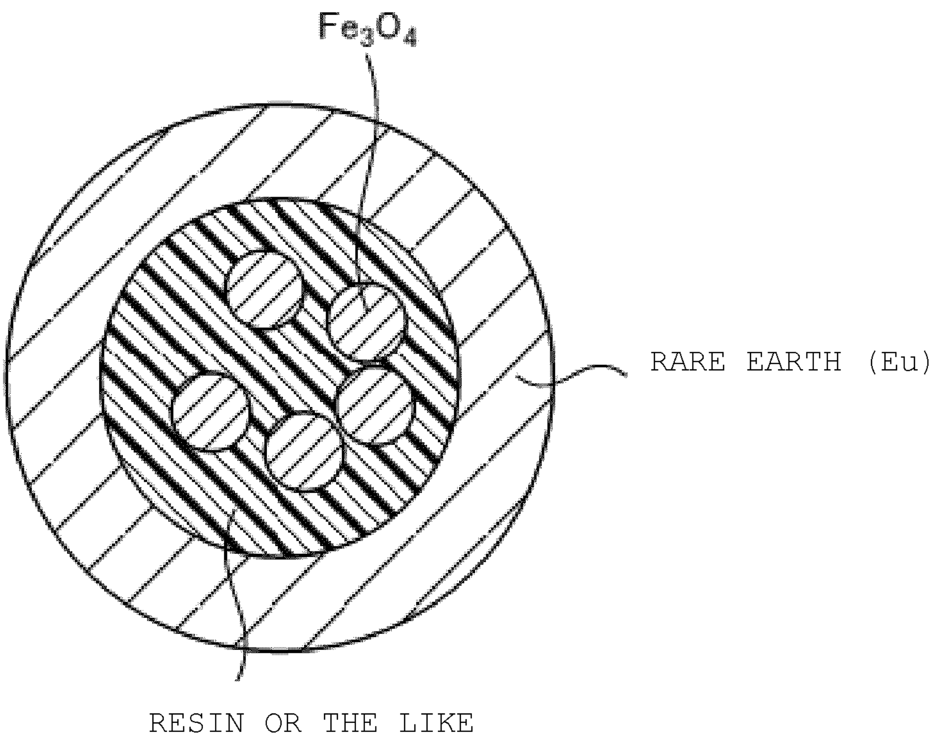

## INTERNATIONAL SEARCH REPORT

International application No. PCT/JP2018/048622

A. CLASSIFICATION OF SUBJECT MATTER

Int.Cl. C08L101/12(2006.01)i, C08F2/12(2006.01)i, C08L25/06(2006.01)i, C09K11/06(2006.01)i, G01N33/533(2006.01)i, G01N33/553(2006.01)i

According to International Patent Classification (IPC) or to both national classification and IPC

B. FIELDS SEARCHED

Minimum documentation searched (classification system followed by classification symbols)

Int.Cl. C08L1/00-101/14, C08F2/00-2/60, C09K11/00-11/89, G01N33/48-33/98, A61K39/00-51/12, C08J3/00-3/28

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)

JSTPlus/JMEDPlus/JST7580 (JDreamIII), CAplus/REGISTRY (STN)

C. DOCUMENTS CONSIDERED TO BE RELEVANT

| Category* | Citation of document, with indication, where appropriate, of the relevant passages | Relevant to claim No. |
|---|---|---|
| Y<br>A | AIKAWA, T. et al., “Polystyrene latex particles containing europium complexes prepared by miniemulsion polymerization using bovine serum albumin as a surfactant for biochemical diagnosis”, Colloids and Surfaces B: Biointerfaces, 2016, vol. 145, no. 1, pp. 152-159, in particular, see abstract, page 152, right column, line 15 to page 153, left column, line 11, fig. 1-8, table 1, etc. | 1-7, 9-12<br>8 |

☒ Further documents are listed in the continuation of Box C. ☐ See patent family annex.

* Special categories of cited documents:
- “A” document defining the general state of the art which is not considered to be of particular relevance
- “E” earlier application or patent but published on or after the international filing date
- “L” document which may throw doubts on priority claim(s) or which is cited to establish the publication date of another citation or other special reason (as specified)
- “O” document referring to an oral disclosure, use, exhibition or other means
- “P” document published prior to the international filing date but later than the priority date claimed
- “T” later document published after the international filing date or priority date and not in conflict with the application but cited to understand the principle or theory underlying the invention
- “X” document of particular relevance; the claimed invention cannot be considered novel or cannot be considered to involve an inventive step when the document is taken alone
- “Y” document of particular relevance; the claimed invention cannot be considered to involve an inventive step when the document is combined with one or more other such documents, such combination being obvious to a person skilled in the art
- “&” document member of the same patent family

| Date of the actual completion of the international search | Date of mailing of the international search report |
|---|---|
| 07 March 2019 (07.03.2019) | 26 March 2019 (26.03.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

INTERNATIONAL SEARCH REPORT

International application No. PCT/JP2018/048622

C (Continuation). DOCUMENTS CONSIDERED TO BE RELEVANT

| Category* | Citation of document, with indication, where appropriate, of the relevant passages | Relevant to claim No. |
|---|---|---|
| Y<br>A | 長谷川美貴 外, 発光性希土類錯体の構造と光化学, SPring-8 Information, 2011, vol. 16, no. 3, pp. 191-196, in particular, see "1. Introduction: What can be done only by rare-earth complexes", fig. 1, etc. (HASEGAWA, Miki et al., "Structure and Photochemistry of Luminescent Rare-Earth Complexes") | 1-7, 9-12<br>8 |
| Y<br>A | HUHTINEN, P. et al., "Synthesis, Characterization, and Application of Eu(III), Tb(III), Sm(III), and Dy(III) Lanthanide Chelate Nanoparticle Labels", Analytical Chemistry, 2005, vol. 77, no. 8, pp. 2643-2648, in particular, see abstract, etc. | 1-7, 9-12<br>8 |
| Y<br>A | JP 2017-7976 A (NATIONAL INSTITUTE OF ADVANCED INDUSTRIAL SCIENCE AND TECHNOLOGY) 12 January 2017, paragraph [0004] (Family: none) | 1-7, 9-12<br>8 |
| Y<br>A | JP 2010-37169 A (KEIO GIJUKU) 18 February 2010, paragraph [0011] (Family: none) | 1-7, 9-12<br>8 |
| A | LI, X. et al., "Synthesis of novel mixed-ligand complexes of lanthanide ions with 1, 4-bis-(1'-pheny1-3'-methy1-5'-pyrazolone-4')-1, 4-butanedione and 1, 10-phenanthroline and their UV, IR, $^{1}H$ NMR, fluorescence and thermal analysis studies", Polyhedron, 1990, vol. 9, no. 18, pp. 2285-2291 | 1-12 |
| A | JP 56-164503 A (RHONE POULENC INDUSTRUES) 17 December 1981 & EP 38730 A1 & US 4358388 A | 1-12 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2005062087 A
- WO 2012173002 A
- JP 2008127454 A
- WO 2010029739 A
- JP 10055911 A
- JP 2010528640 A
- JP 5574492 B
- JP 59500691 A


### Non-patent literature cited in the description


- **TOSHINAO NAKATSU.** Secchaku (Adhesion). *Koubunshikankoukai (polymer publication society),* 1996, vol. 40 (8), 342-350
- Kousomenekisokuteihou (Enzyme immunoassay). IGAKU-SHOIN Ltd, 1982
- **YUICHI YAMAMURA.** Ikagakujikkenkouza (Medical chemistry experiment course). Nakayama Shoten Co., Ltd, 1971, vol. 8
- **AKIRA KAWAOI.** Zusetsu keikoukoutai (Illustrated fluorescent antibodies). Soft Science Co., Ltd, 1983